# EUROPÄISCHE PATENTSCHRIFT

(11) **EP 1 078 029 B1**
(45) Veröffentlichungstag und Bekanntmachung des Hinweises auf die Patenterteilung: **06.03.2002**
(21) Anmeldenummer: 99923505.4
(22) Anmeldetag: 03.05.1999
(51) Int. Cl.: C11D 1/14, C11D 17/06

(54) **ALKYLSULFAT-GRANULATE**
ALKYL SULFATE GRANULATES
GRANULES DE SULFATE D'ALKYLE

(30) Priorität: 11.05.1998 DE 19820943
(43) Veröffentlichungstag der Anmeldung: 28.02.2001
(73) Patentinhaber: Cognis Deutschland GmbH, 40589 Düsseldorf (DE)
(72) Erfinder: ASSMANN, Georg, D-41363 Jüchen (DE); BLOCHWITZ, Olaf, D-39307 Genthin (DE); SYLDATH, Andreas, D-40789 Monheim (DE); KISCHKEL, Ditmar, D-40789 Monheim (DE)
(86) Internationale Anmeldenummer: EP9902967
(87) Internationale Veröffentlichungsnummer: WO9958630

(56) Entgegenhaltungen:
- EP-A- 0 665 288
- WO-A-93/04162
- WO-A-95/02036
- WO-A-95/08616
- WO-A-95/29981
- WO-A-96/00611
- US-A- 4 253 993

## Beschreibung

Die Erfindung betrifft ein Verfahren zur Herstellung geruchsamer Granulate von Alkylsulfaten sowie deren Verwendung zur Herstellung von Wasch-, Spül- und Reinigungsmitteln sowie zur Herstellung reinigender Kosmetika.

Alkylsulfate sind gängige Tenside, die ihre Verwendung meist in Wasch- und Reinigungsmitteln finden. Durch die Herstellung bedingt, fallen sie als wäßrige Pasten mit Wassergehalten von 35 bis 65 Gew.% an. Um feste und rieselfähige Produkte zu erhalten, werden die wäßrigen Pasten beispielsweise im Sprühturm getrocknet, wobei sprühgetrocknete Pulver entstehen, die jedoch nur ein geringes Schüttgewicht aufweisen. Eine Alternative zur Sprühtrocknung stellt die Granulierung, insbesondere in der Wirbelschicht, dar. So ist beispielsweise aus der Europäischen Patentschrift **EP-B-603 207** bekannt, daß wäßrige Pasten von Alkylsulfaten in der kontinuierlichen Wirbelschicht durch Granulierung und gleichzeitiger Trocknung in Granulate mit hohem Schüttgewicht überführt werden können. Nach diesem Verfahren ist es auch möglich, anorganische oder organische Trägermaterialien zuzumischen.

Obgleich es also prinzipiell bekannt ist, aus wäßrigen Pasten von Alkylsulfaten Granulate herzustellen, ist das Problem des unangenehmen Geruches wäßriger Alkylsulfatpasten sowie sprühgetrockneter Pulver oder Granulate daraus noch nicht gelöst. Dieser unangenehme Geruch tritt vor allem bei solchen Alkylsulfaten auf, die 8 bis 14 Kohlenstoffatome im Alkylrest aufweisen. Aufgrund dieses unangenehmen Geruches konnten derartige Alkylsulfate bislang nur in geringen Mengen in der Kosmetik eingesetzt werden und auch nur dann, wenn sie in hohen Mengen parfümiert waren.

Die Aufgabe der vorliegenden Erfindung bestand in der Bereitstellung von rieselfähigen, staubfreien und vor allem geruchsarmen Alkylsulfaten.

Überraschenderweise konnte die Aufgabe gelöst werden, wenn man die auf herkömmlichem Wege zugänglichen wäßrigen Alkylsulfat-Pasten in der Wirbelschicht solange granuliert und gleichzeitig trocknet, bis der Gehalt an unsulfiertem Anteil in den Granulaten unter 0,2 Gew.% - bezogen auf Aktivsubstanzgehalt - sank.

Ein Gegenstand der vorliegenden Erfindung betrifft daher geruchsarme Granulate von Alkylsulfaten der Formel **(I),**

R-O-SO₃X **(I)**

in der R für einen Alkylrest mit 6 bis 18 Kohlenstoffatomen und
X für ein Alkali-, Erdalkali-, Ammonium-, Alkylammonium-, Alkanolammonium- oder Glucammonium-Ion steht,
mit unsulfierten Anteilen unter 0,2 Gew.% - bezogen auf Aktivsubstanzgehalt.

Der Rest R in Formel **(I)** leitet sich ab von Alkoholen der Formel ROH, wobei R die obige Bedeutung hat. R kann sich demgemäß von den Alkoholen wie Capronalkohol, Caprylalkohol. 2-Ethylhexylalkohol, Caprinalkohol, Laurylalkohol, Isotridecylalkohot, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylaikohol. Elaeostearylalkohol, Arachylalkohol, Gadoleylalkohol. Behenylalkohol, Erucylalkohol und Brassidylalkohol sowie deren technische Mischungen, die z.B. bei der Hochdruckhydrierung von technischen Methylestern auf Basis von Fetten und Ölen entstehen, ableiten. Bevorzugt leitet sich R ab von Alkoholen mit 8 bis 14 Kohlenstoffatomen und insbesondere von technischen Mischungen von derartigen Alkoholen. Insbesondere leitet sich R ab von technischen Mischungen des Octanols, so daß sich für R in der Formel **(I)** insbesondere folgende Zusammensetzung der Alkylreste ergibt:
0 bis 10 Gew.% Alkylreste mit 6 Kohlenstoffatomen
50 bis 100 Gew.% Alkylreste mit 8 Kohlenstoffatomen
0 bis 10 Gew.% Alkylreste mit 10 Kohlenstoffatomen
0 bis 10 Gew.% Alkylreste mit 12 Kohlenstoffatomen
0 bis 10 Gew.% Alkylreste mit 14 Kohlenstoffatomen
0 bis 10 Gew.% Alkylreste mit 16 Kohlenstoffatomen
0 bis 10 Gew.% Alkylreste mit 18 Kohlenstoffatomen

Ein weiterer Gegenstand der vorliegenden Erfindung betrifft ein Verfahren zur Herstellung geruchsamer Granulate von Alkylsulfaten der Formel **(I)** mit unsulfierten Anteilen unter 0,2 Gew.% - bezogen auf Aktivsubstanzgehalt -, wobei man wäßrige Pasten der Alkylsulfate mit einem Gehalt an unsulfierten Anteilen über 0,3 Gew.% ggf. in Anwesenheit von Trägermaterialien in der Wirbelschicht granuliert und gleichzeitig trocknet.

Im Sinne der vorliegenden Erfindung bedeutet der Begriff "Aktivsubstanzgehalt" der Gehalt an nach Epton waschaktivem Alkylsulfat.

Nach dem erfindungsgemäßen Verfahren werden herkömmliche wäßrige Alkylsulfat-Pasten eingesetzt, wie sie nach der Sulfatierung der entsprechenden Alkohole und anschließender Neutralisation erhalten werden. Da die Sulfatierung, vor allem in großtechnischen Anlagen, nicht vollständig verläuft und zum Teil auch nicht näher charakterisierte Nebenprodukte ohne Sulfatgruppen liefert, enthalten die herkömmlichen Alkylsulfat-Pasten auch stets sogenannte unsulfierte Anteile. Unter diesen unsulfierten Anteilen werden die nicht umgesetzten Alkohole als auch die Nebenprodukte verstanden. Üblicherweise enthalten die wäßrigen Alkylsulfat-Pasten die unsulfierten Anteile in Mengen über 0,3 Gew.%, meist zwischen 0,5 und 2,5 Gew.% - bezogen auf Aktivsubstanzgehalt. So enthalten beispielsweise wäßrige technische Octylsulfat-Pasten zwischen 0,5 und 2,5 Gew.% unsulfierte Anteile sowie 35 bis 65 Gew.%, vorzugsweise 35 bis 45 Gew.%, technische Octylsulfate, mit der oben angegebenen Kettenverteilung für den technischen Octylrest, als auch in untergeordneten Mengen, meist unter 3 Gew.%, Alkalisalze wie Natriumchlorid und/oder Natriumsulfat. Der zu 100 Gew.% fehlende Rest der Pasten ist Wasser.

Diese herkömmlichen Alkylsulfat-Pasten können alleine oder unter Zumischung von festen Trägermaterialien in der Wirbelschicht granuliert und gleichzeitig getrocknet werden. In der Wirbelschicht verdampft das Wasser aus der Paste, wodurch angetrocknete bis getrocknete Keime entstehen, die mit weiteren eingebrachten wäßrigen Pasten bzw. mit den zugemischten Trägermaterialien umhüllt, granuliert und wiederum gleichzeitig getrocknet werden.

Im einfachsten Fall werden sie alleine in der Wirbelschicht granuliert und gleichzeitig getrocknet. Dabei werden die wäßrigen Pasten gleichzeitig oder nacheinander über eine oder mehrere Düsen eingebracht. Sofern Trägermaterialien zugemischt werden sollen, werden diese gleichzeitig mit den wäßrigen Pasten, aber separat von diesen, vorzugsweise über eine automatisch geregelte Feststoffdosierung zugemischt.

Bevorzugt eingesetzte Wirbelschichtapparate besitzen Bodenplatten mit einem Durchmesser zwischen 0,4 und 5 m, beispielsweise 1,2 m oder 2,5 m. Als Bodenplatte können Lochbodenplatten, eine Conidurplatte (Handelsprodukt der Firma Hein& Lehmann, Bundesrepublik Deutschland) oder Lochbodenplatten eingesetzt werden, deren Löcher (Durchtrittsöffnungen) von einem Gitternetz mit Maschenweiten kleiner als 600 um bedeckt sind. Dabei kann das Gitternetz innerhalb oder oberhalb der Durchtrittsöffnungen angeordnet sein. Vorzugsweise liegt das Gitternetz jedoch unmittelbar unterhalb der Durchtrittsöffnungen des Anströmbodens. Vorteilhafterweise ist dies so realisiert, daß eine Metall-Gaze mit der entsprechenden Maschenweite aufgesintert ist. Vorzugsweise besteht die Metallgaze aus dem gleichen Material wie der Anströmboden, insbesondere aus Edelstahl. Vorzugsweise liegt die Maschenweite des genannten Gitternetzes zwischen 200 und 400 µm.

Bevorzugt im Sinne der Erfindung wird das Verfahren bei Wirbelluftgeschwindigkeiten zwischen 1 und 8 m/s und insbesondere zwischen 1,5 und 5,5 m/s durchgeführt. Der Austrag der Granulate erfolgt vorteilhafterweise über eine Größenklassierung der Granulate. Diese Klassierung erfolgt bevorzugt mittels einem entgegengeführtem Luftstrom (Sichterluft), der so reguliert wird, daß erst Teilchen ab einer bestimmten Teilchengröße aus der Wirbelschicht entfernt und kleinere Teilchen in der Wirbelschicht zurückgehalten werden. In einer bevorzugten Ausführungsform setzt sich die einströmende Luft aus der beheizten oder unbeheizten Sichterluft und der beheizten Bodeniuft zusammen. Die Bodenlufttemperatur liegt dabei vorzugsweise zwischen 80 und 400 °C. Die Wirbelluft kühlt sich durch Wärmeverluste und durch die Verdampfungswärme ab und beträgt vorzugsweise etwa 5 cm oberhalb der Bodenplatte 60 bis 120 °C, vorzugsweise 60 bis 90 °C und insbesondere 60 bis 85 °C. Die Luftaustrittstemperatur liegt vorzugsweise zwischen 60 und 120 °C, insbesondere unterhalb 80 °C. Weitere vorteilhafte Ausgestaltungen des erfindungsgemäßen Verfahrens finden sich in der zur Zeit noch unveröffentlichten deutschen Patentanmeldung P **197 504 24.8** sowie in der bereits zitierten europäischen Patentschrift **EP-B-603 207.**

Bei dem bevorzugt durchgeführtem Verfahren in der Wirbelschicht ist es notwendig, daß zu Beginn des Verfahrens eine Startmasse vorhanden ist, die als anfänglicher Träger für die eingesprühte Tensidpaste dient. Als Startmasse eignen sich die ggf. Trägermaterialien enthaltenen Alkylsulfat-Granulate selber, die bereits bei einem vorangegangenen Verfahrensablauf erhalten wurden oder die Trägermaterialien können als Startmasse eingesetzt werden. Insbesondere werden Alkylsulfat-Granulate mit einer Korngröße im Bereich über 0,2 und unter 0,9 mm als Startmasse eingesetzt und vorzugsweise über einen Walzenstuhl eingespeist.

Sofern im Sinne der Erfindung Trägermaterialien zugemischt werden, handelt es sich vorzugsweise um anorganische Trägermaterialien und insbesondere um Alkalicarbonate, Alkalisulfate, kristalline oder amorphe Alkalisilikate, kristalline oder amorphe Schichtsilikate und/oder Zeolithe. Der Anteil an Trägermaterialien zu Alkylsulfat ist nicht kritisch und kann im Gewichtsverhältnis zwischen 0 : 100 bis 50 : 50, vorzugsweise bis 20 : 80 liegen.

Bevorzugt werden die aus der Wirbelschicht erhaltenen Granulate anschließend in einem seperaten Wirbelbett abgekühlt und mittels eines Siebes klassiert in Granulate mit Korngrößen zwischen 0,9 und 5 mm als Gutkomfraktion, in Granulate über 5 mm als Überkornfraktion und in Granulate unter 0.9 mm als Unterkornfraktion. Die Granulate der Unterkornfraktion werden wieder in die Wirbelschicht zurückgeführt. Die Überkornfraktion wird gemahlen, vorzugsweise in Korngrößen unter 0,9 mm, und ebenfalls in die Wirbelschicht zurückgeführt.

Im Sinne der vorliegenden Erfindung werden die Tensidgranulate als getrocknet betrachtet, sofern der Gehalt an freiem Wasser unter 10 Gew.%, vorzugsweise von 0,1 bis 2 Gew.%, jeweils bezogen auf die fertigen Granulate, beträgt.

Erfindungswesentlich für das erfindungsgemäße Verfahren ist, daß die Granulate solange in der Wirbelschicht verbleiben, bis der Anteil an Unsulfiertem unter 0,2 Gew.% - bezogen auf Aktivsubstanz - liegt. Vorzugsweise wird das Verfahren solange durchgeführt, bis der Gehalt an Unsulfiertem im Bereich von 0,08 bis 0,15 Gew.% - bezogen auf Aktivsubstanz - liegt. Dies wird unter den obigen Bedingungen in der Regel bei einer Verweilzeit von 1 bis 5 Stunden, vorzugsweise zwischen 1,5 bis 3 Stunden, der Granulate in der Wirbelschicht erreicht. Wie es zu einer Reduzierung des Gehaltes an Unsulfiertem kommt, ist zur Zeit noch nicht vollständig geklärt. Die Anmelderin geht aber davon aus, daß durch die Trocknung in der Wirbelschicht Anteile an Unsulfiertem "mitgerissen" werden und somit aus dem Granulat entfernt werden.

Nach dem erfindungsgemäßen Verfahren werden Granulate mit hohem Schüttgewicht erhalten. Der Aktivsubstanzgehalt schwankt je nach Zusatz von eingesetztem Trägermaterialien und liegt insbesondere über 80 Gew.%, vorzugsweise zwischen 85 und 95 Gew.%.

Die nach dem erfindungsgemäßen Verfahren erhaltenen Alkylsulfat-Granulate sind geruchsarm, sowohl als Granulate als auch nach Auflösen der Granulate in Wasser als wäßrige Lösung. Der Geruch ist deutlich weniger intensiv als bei den eingesetzten Alkylsulfat-Pasten und ebenfalls geringer als bei sprühgetrockneten Pulvern. Des weiteren sind die Alkylsulfat-Granulate staubfrei und rieselfähig und nicht bzw. kaum klebrig, so daß sie gut in big bags handhabbar sind.

Ein weiterer Gegenstand der vorliegenden Erfindung betrifft die Verwendung der erfindungsgemäß hergestellten Granulate von Alkylsulfaten nach Formel **(I)** als Tenside zur Herstellung von Wasch-, Spül- und Reinigungsmitteln. In den Wasch-, Spül- und Reinigungsmitteln können die erfindungsgemäßen Granulate in üblichen Mengen, vorzugsweise im Bereich von 0,1 bis 30 Gew.% - bezogen auf Mittel - enthalten sein.

Ein weiterer Gegenstand der vorliegenden Erfindung betrifft die Verwendung der erfindungsgemäß hergestellten Granulate von Alkylsulfaten nach Formel **(I)** als Tenside zur Herstellung reinigender Kosmetika für Haare und Haut, insbesondere für die Herstellung von Haarshampoos. Duschbäder. Waschlotionen und dergleichen. In diesen Mitteln können die erfindungsgemäßen Granulate in Mengen von 0,1 bis 30 Gew.% - bezogen auf Mittel - enthalten sein.

### Beispiele

### I. Einsatzstoffe

A1) Octanolsulfat, Natriumsalz, in Form einer 43,9 gew.%igen wäßrigen Paste an Octanolsulfat. Kenndaten: Kettenverteilung: 0 - 2 Gew.% C₆; 93 - 100 Gew.% C₈; 0 - 5 Gew.% C₁₀; Unsulfiertes 1,0 Gew.% - bezogen auf 43,9 Gew.% Aktivsubstanzgehalt-; Natriumsulfat: 0,5 Gew.%; Natriumchlorid 0,1 Gew.%.

### II. Herstellung der Granulate in der Wirbelschicht

Die wäßrige Paste A1) wurde in die Wirbelschichtanlage eingedüst.

| Kenndaten des Verfahrens: | |
|---|---|
| Zuluftmenge | 17 000 bis 20 000 Nm/h |
| Zulufttemperatur | 170°C |
| Luftaustrittstemperatur | 68-71 °C |
| Verdüsungsmenge | 1000 - 1200 kg/h |
| Wirbelschichtdifferenzdruck | größer 30 mbar |
| Dauer des Versuches | 15 h |
| Produktmenge | 6,5 t |
| Durchschnittliche Verweilzeit | 1,7 Stunden |

| Es wurden Octanolsulfat-Granulate erhalten mit folgenden Kenndaten: | |
|---|---|
| Schüttgewicht | 710 - 740 g/l |
| Aktivsubstanzgehalt | 88 - 95 g/l |
| Kettenverteilung | 0 - 2 Gew.% C₆; 93 - 100 Gew.% C₈; 0 - 5 Gew.% C₁₀ |
| Unsulfiertes | 0.17 Gew.%- bezogen auf Aktivsubstanzgehalt- |
| Geruch | schwach |

### III. Geruch

Es wurden 5 gew.%ige wäßrige Lösungen hergesellt aus
■ eingesetzter Paste in A1)
■ Pulver, erhalten durch Sprühtrocknung von Paste in A1)
■ erfindungsgemäßem Granulat

Ein subjektiver Vergleich des Geruches ergab einen sehr starken Geruch für die Paste, einen deutlichen Geruch des sprühgetrockneten Pulvers und einen sehr schwachen Geruch beim erfindungsgemäßen Granulat.

## Patentansprüche

1. Geruchsarme Granulate von Alkylsulfaten der Formel **(I)**,
R-O-SO₃X **(I)**
in der R für einen Alkylrest mit 6 bis 18 Kohlenstoffatomen und
X für ein Alkali-, Erdalkali-, Ammonium-, Alkylammonium-, Alkanolammonium- oder Glucammonium-Ion steht,
mit unsulfierten Anteilen unter 0,2 Gew.% - bezogen auf Aktivsubstanzgehalt.

2. Verfahren zur Herstellung geruchsamer Granulate von Alkylsulfaten der Formel **(I),**
R-O-SO₃X **(I)**
in der R für einen Alkylrest mit 6 bis 18 Kohlenstoffatomen und
X für ein Alkali-, Erdalkali-, Ammonium-, Alkylammonium-, Alkanolammonium- oder Glucammonium-Ion steht,
mit unsulfierten Anteilen unter 0,2 Gew.% - bezogen auf Aktivsubstanzgehalt -, wobei man wäßrige Pasten der Alkylsulfate mit einem Gehalt an unsulfierten Anteilen über 0,3 Gew.% ggf. in Anwesenheit von Trägermaterialien in der Wirbelschicht granuliert und gleichzeitig trocknet.

3. Verfahren nach Anspruch 2, **dadurch gekennzeichnet, daß** Granulate hergestellt werden, die einen Aktivsubstanzgehalt an Alkylsulfaten über 80 Gew.%, vorzugsweise zwischen 85 und 95 Gew.% aufweisen.

4. Verfahren nach einem der Ansprüche 2 oder 3, **dadurch gekennzeichnet, daß** Granulate der Formel **(I)** hergestellt werden, in der R für Alkylreste der Zusammensetzung
0 bis 10 Gew.% Alkylreste mit 6 Kohlenstoffatomen
50 bis 100 Gew.%. Alkylreste mit 8 Kohlenstoffatomen
0 bis 10 Gew.% Alkylreste mit 10 Kohlenstoffatomen
0 bis 10 Gew.% Alkylreste mit 12 Kohlenstoffatomen
0 bis 10 Gew.% Alkylreste mit 14 Kohlenstoffatomen
0 bis 10 Gew.% Alkylreste mit 16 Kohlenstoffatomen
0 bis 10 Gew.% Alkylreste mit 18 Kohlenstoffatomen
steht.

5. Verfahren nach einem der Ansprüche 2 bis 4, **dadurch gekennzeichnet, daß** man wäßrige Pasten von Alkylsulfaten mit einem unsulfierten Anteil von 0,5 bis 2,5 Gew.% - bezogen auf Aktivsubstanzgehalt- einsetzt.

6. Verfahren nach einem der Ansprüche 2 bis 5, **dadurch gekennzeichnet, daß** man als Trägermaterialien Alkalicarbonate, Alkalisulfate, kristalline oder amorphe Alkalisilikate oder Schichtsilikate und/oder Zeolithe zumischt.

7. Verfahren nach einem der Ansprüche 2 bis 6, **dadurch gekennzeichnet, daß** die Verweilzeit der Granulate in der Wirbelschicht etwa 1 bis 5 Stunden, vorzugsweise 1,5 bis 3 Stunden beträgt.

8. Verwendung der Granulate von Alkylsulfaten nach Anspruch 1 als Tenside zur Herstellung von Wasch-, Spül- und Reinigungsmitteln.

9. Verwendung der Granulate von Alkylsulfaten nach Anspruch 1 als Tenside zur Herstellung reinigender Kosmetika für Haare und Haut.

## Claims

1. Low-odour granules of alkyl sulfates corresponding to formula (I):
R-O-SO₃X (I)
in which R is an alkyl group containing 6 to 18 carbon atoms and
X is an alkali metal, alkaline earth metal, ammonium, alkyl ammonium, alkanolammonium or glucammonium ion,
with unsulfonated components of less than 0.2% by weight, based on active substance content.

2. A process for the production of low-odour granules of alkyl sulfates corresponding to formula (I):
R-O-SO₃X (I)
in which R is an alkyl group containing 6 to 18 carbon atoms and
X is an alkali metal, alkaline earth metal, ammonium, alkyl ammonium, alkanolammonium or glucammonium ion,
with unsulfonated components of less than 0.2% by weight, based on active substance content,
**characterized in that** aqueous pastes of the alkyl sulfates containing more than 0.5% by weight unsulfonated components are granulated and at the same dried in a fluidized bed, optionally in the presence of carrier materials.

3. A process as claimed in claim 2, **characterized in that** granules with an active substance content of alkyl sulfates of more than 80% by weight and preferably between 85 and 95% by weight are produced.

4. A process as claimed in claim 2 or 3, **characterized in that** granules corresponding to formula (I) where R represents alkyl groups with the following composition:
0 to 10% by weight C₆ alkyl groups,
50 to 100% by weight C₈ alkyl groups,
0 to 10% by weight C₁₀ alkyl groups,
0 to 10% by weight C₁₂ alkyl groups,
0 to 10% by weight C₁₄ alkyl groups,
0 to 10% by weight C₁₆ alkyl groups,
0 to 10% by weight C₁₈ alkyl groups,
are produced.

5. A process as claimed in any of claims 2 to 4, **characterized in that** aqueous pastes of alkyl sulfates with an unsulfonated component of 0.5 to 2.5% by weight, based on active substance content, are used.

6. A process as claimed in any of claims 2 to 5, **characterized in that** alkali metal carbonates, alkali metal sulfates, crystalline or amorphous alkali metal silicates or layer silicates and/or zeolites are used as the carrier materials.

7. A process as claimed in any of claims 2 to 6, **characterized in that** the residence time of the granules in the fluidized bed is about 1 to 5 hours and preferably 1.5 to 3 hours.

8. The use of the granules of alkyl sulfates claimed in claim 1 as surfactants for the production of laundry detergents, dishwashing detergents and cleaners.

9. The use of the granules of alkyl sulfates claimed in claim 1 as surfactants for the production of cosmetics for cleaning the hair and skin.

## Revendications

1. Granulés à faible odeur de sulfates d'alkyle de formule (I),
R-O-SO₃X (I)
dans laquelle R représente un reste alkyle ayant de 6 à 18 atomes de carbone, et X représente un ion de métal alcalin, alcalino-terreux, d'ammonium, d'alkylammonium, d'alcanolammonium ou de glucammonium,
ayant des fractions non sulfatées en dessous de 0,2 % en poids, rapporté à la teneur en substance active.

2. Procédé de production de granulés à faible odeur de sulfates d'alkyle de formule (I),
R-O-SO₃X (I)
dans laquelle R représente un reste alkyle ayant de 6 à 18 atomes de carbone, et X représente un ion de métal alcalin, alcalino-terreux, d'ammonium, d'alkylammonium, d'alcanolammonium ou de glucammonium,
ayant des fractions non sulfatées en dessous de 0,2 % en poids, rapporté à la teneur en substance active, dans lequel on granule des pâtes aqueuses des sulfates d'alkyle ayant une teneur en fractions non sulfatées supérieure à 0,3 % en poids, éventuellement en présence de matériaux de support, en lit fluidifié, et en même temps on sèche.

3. Procédé selon la revendication 2,
**caractérisé en ce qu'**
on produit des granulés qui possèdent une teneur en substance active en sulfates d'alkyle, supérieure à 80 % en poids, de préférence entre 85 et 95 % en poids.

4. Procédé selon l'une des revendications 2 ou 3,
**caractérisé en ce qu'**
on produit des granulés de formule (I) dans laquelle R représente des restes alkyle de composition :
de 0 à 10 % en poids de restes alkyle ayant 6 atomes de carbone,
de 50 à 100 % en poids de restes alkyle ayant 8 atomes de carbone,
de 0 à 10 % en poids de restes alkyle ayant 10 atomes de carbone,
de 0 à 10 % en poids de restes alkyle ayant 12 atomes de carbone,
de 0 à 10 % en poids de restes alkyle ayant 14 atomes de carbone
dé 0 à 10 % en poids de restes alkyle ayant 16 atomes de carbone
de 0 à 10 % en poids de restes alkyle ayant 18 atomes de carbone.

5. Procédé selon l'une quelconque des revendications 2 à 4,
**caractérisé en ce qu'**
on met en oeuvre des pâtes aqueuses de sulfates d'alkyle ayant une fraction non sulfatée de 0,5 à 2,5 % en poids rapporté à la teneur en substance active.

6. Procédé selon l'une quelconque des revendications 2 à 5,
**caractérisé en ce qu'**
on ajoute par mélange en tant que matériau de support, des carbonates de métal alcalin, des sulfates de métal alcalin, des silicates de métal alcalin cristallins ou amorphes ou des silicates lamellaires et/ou des zéolithes.

7. Procédé selon l'une quelconque des revendications 2 à 6,
**caractérisé en ce qu'**
le temps de séjour des granulés en lit fluidifié s'élève à environ 1 à 5 heures, de préférence de 1,5 à 3 heures.

8. Utilisation des granulés de sulfates d'alkyle selon la revendication 1, comme agents tensioactifs pour la préparation de produits de lavage, de rinçage et de nettoyage.

9. Utilisation des granulés de sulfates d'alkyle selon la revendication 1, comme agents tensioactifs pour la préparation de cosmétiques nettoyants pour les cheveux et la peau.
